Europäisches Patentamt

European Patent Office

Office européen des brevets

(19)

(11) Veröffentlichungsnummer : **0 058 745**
**B1**

(12) **EUROPÄISCHE PATENTSCHRIFT**

(45) Veröffentlichungstag der Patentschrift :
18.09.85

(51) Int. Cl.⁴ : **A 61 F 2/32**

(21) Anmeldenummer : **81106950.9**

(22) Anmeldetag : **04.09.81**

(54) Gerader, blattartiger Schaft für eine Gelenkendoprothese.

(30) Priorität : 19.02.81 CH 1091/81

(43) Veröffentlichungstag der Anmeldung :
01.09.82 Patentblatt 82/35

(45) Bekanntmachung des Hinweises auf die Patenterteilung : 18.09.85 Patentblatt 85/38

(84) Benannte Vertragsstaaten :
**AT DE FR GB IT NL**

(56) Entgegenhaltungen :
EP-A- 0 032 165
CH-A- 587 653
CH-C- 560 042
DE-C- 837 294

(73) Patentinhaber : **GEBRÜDER SULZER AKTIENGE-SELLSCHAFT**
**Zürcherstrasse 9**
**CH-8401 Winterthur (CH)**

**Protek AG**
**Stadtbachstrasse 64**
**CH-3001 Bern (CH)**

(72) Erfinder : **Niederer, Peter Gino, Dipl.-Ing.**
**Reichenbachstrasse 6**
**CH-3052 Zollikofen (CH)**
Erfinder : **Frey, Otto**
**Walrütistrasse 56**
**CH-8400 Winterthur (CH)**

(74) Vertreter : **Dipl.-Ing. H. Marsch Dipl.-Ing. K. Sparing**
**Dipl.-Phys.Dr. W.H. Röhl Patentanwälte**
**Rethelstrasse 123**
**D-4000 Düsseldorf (DE)**

## Beschreibung

Die Erfindung betrifft einen geraden, blattartigen Schaft für eine Gelenkendoprothese, insbesondere eine Hüftgelenkprothese, dessen Blatt sich vom distalen freien Ende zunächst symmetrisch zu einer Längsmittelachse allseitig konisch erweitert, wobei der Konus der beiden Schmalseiten und die laterale Erweiterung der Blattseiten auf etwa 3/4 der Schafthöhe enden, während medial die Erweiterung der Blattseiten aus dem Konus heraus in einer stetig gekrümmten Kurve in einen, das Schaftblatt vom Prothesenhals trennenden Kragen mündet.

Prothesenschäfte der genannten Art, die vor allem für die Verankerung des Femurteils von Hüftgelenkprothesen verwendet werden, sind beispielsweise aus der EP-A-0 032 165 bekannt ; im distalen konischen Teil sind in die Blattseiten zwei relativ breite und tiefe Längsnuten eingelassen, durch die einerseits bei unveränderter Festigkeit das implantierte Fremdmaterial verringert und andererseits die Oberfläche für das Anhaften von Gewebe oder Knochenzement vergrössert werden.

Die Wirkung dieser sogenannten Geradschäfte beruht zu einem wesentlichen Teil darauf, dass er sich in einem auf seinen Dimensionen abgestimmten Hohlraum der Markhöhle verklemmt, wodurch ein ihn gegebenenfalls umschliessender Köcher aus Knochenzement weitgehend von tragenden Funktionen entlastet wird ; die tragende Abstützung der Prothese erfolgt dann in erster Linie durch Verklemmen und Anpassen der stetig gekrümmten Kurve der medialen Schmalseite auf dem im Femur medial gelegenen Calcarbogen. Diese Schäfte sind darüberhinaus dauernd Belastungen unterworfen, die eine Rotation des Schaftes zu bewirken suchen.

Weiterhin ist ein gerader, zylindrischer Schaft bekannt (DE-C-837 294), dessen distales Ende in einer konischen Spitze ausläuft ; die Mantelfläche des Kreiszylinders ist auf dem ganzen Umfang mit Rillen versehen, die parallel zur Zylinderachse und zur Mantelfläche verlaufen. Gegen unbeabsichtigte Rotationen im Knochen ist dieser Schaft nur ungenügend gesichert. Weiterhin erfolgt eine Kraftübertragung auf den Knochen bei ihm im wesentlichen in Richtung der Zylinderachse.

Aus der klinischen Praxis heraus hat sich die Forderung ergeben, die Haftung der erwähnten Geradschäfte, die entweder zementfrei in den Hohlraum eingeschlagen oder in einen Knochenzementköcher eingetrieben werden, zu verbessern ; denn es kommt beispielsweise bei einer Fixierung des Schaftes mit Hilfe eines Zementköchers in Umfangsrichtung besonders im Bereich der medialen Schmalseite während der Implantationsoperation zu einer Verdrängung des an sich peripher geschlossenen Zementbettes. An den bisherigen Schäften kann der noch weiche Zement dabei relativ leicht entlanggleiten. Bei den erwähnten Rotationsbelastungen treten darüberhinaus relativ hohe Scherkräfte auf.

Erfindungsgemäss wird die Aufgabe, die Haftung zwischen der Oberfläche derartiger Geradschäfte und dem Knochen bzw. dem Zementbett sowie die Ueberleitung der Kräfte auf den Knochen zu verbessern, dadurch gelöst, dass die Blattseiten des Schaftes im Bereich des Konus mindestens teilweise mit parallel zu Längsmittelachse und zur Oberfläche der Blattseiten verlaufenden Rillen bedeckt sind, während die Schmalseiten glatte Oberflächen haben, wobei der Rillenabstand 0,5 bis 2 mm und die Rillentiefe 0,2 bis 0,5 mm betragen.

Durch derartige Rillen werden nicht nur das geschilderte Fliessen in Umfangsrichtung des Knochenzements bzw. — bei zementfreier Verankerung — dasjenige von durch das Einschlagen entstehenden Trümmerstückchen des spongiosen Knochengewebes behindert und erschwert, sondern auch die Tendenz zum Rotieren gehemmt. Ein konischer, d. h. zu den Blattseiten paralleler Verlauf der Rillen bewirkt, dass die von der Prothese auf den Knochen zu übertragenden Kräfte über die Höhe des Schaftkonus verteilt werden und « nach der Seite » in den Zement bzw. in den Knochen fliessen.

Weiterhin bewirken während des axialen Einsetzens des Schaftes unvermeidliche seitliche Schwankungs- und/oder Rotationsbewegungen geringe örtliche Aufweitungen des Zementbettes, die dazu führen, dass — da der Knochenzement plastisch und nicht elastisch ist — im Bereich dieser Aufweitungen kein Anhaften des Zementes am Schhaft erfolgt. Durch den mit der Blattoberfläche konischen Verlauf der Rillen werden diese Haftungsfehlstellen jedoch durch örtliches Verdrängen von Knochenzement wieder beseitigt.

Die beanspruchten Abmessungen haben sich experimentell als optimal für das Eindringen von Knochenzement oder für das Eindringen, Verdichten und Einwachsen von spongiösem Gewebe und damit für eine innige « Verzahnung » zwischen Implantat und Umgebung ergeben.

Darüberhinaus wird durch die Strukturierung der Blattseiten-Oberflächen die dem Anhaften zur Verfügung stehende Fläche vergrössert, was bekanntlich die Haftfähigkeit des Schaftes gegenüber dem Knochen oder dem Knochenzement verbessert.

Im folgenden wird die Erfindung anhand eines Ausführungsbeispiels im Zusammenhang mit der Zeichnung näher erläutert.

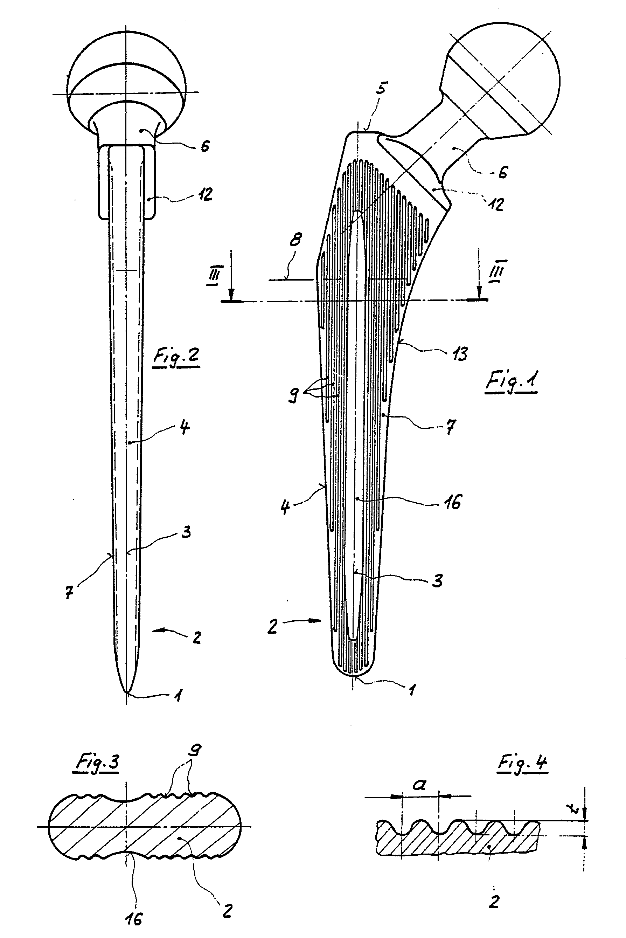

. Figur 1 zeigt am Beispiel einer Hüftgelenkprothese den neuen Prothesenschaft in einer Aufsicht auf das Schaftblatt ;

Figur 2 ist eine Ansicht von Figur 1 von links ;

Figur 3 gibt in grösserem Massstab den Schnitt III-III von Figur 1 wieder, während

Figur 4 schliesslich, gegenüber Figur 3 nochmals vergrössert, ein Detail aus Figur 3 darstellt.

Vom distalen Ende aus erweitert sich der blattartige Schaft 2 zunächst symmetrisch zu seiner Längsmittelachse 3 konisch. Bei der gezeigten Ausführungsform besitzt die laterale Schmalseite 4 des Schaftes 2 eine Unstetigkeit, an der der sich vom distalen Ende 1 erweiternde Konus in eine zur Längsmittelachse 3 hin verlaufende Schrägfläche übergeht. Diese endet in einer mindestens nahezu horizontalen Schulter 5, die den Uebergang zum Schenkelhals 6 bildet.

Der sich erweiternde Konus der medialen Schmalseite 13 des Schaftblattes 7 geht etwa auf halber Schafthöhe in einen Kreisbogen über, der stufenlos in einen den Schaft 2 vom Prothesenhals 6 trennenden kragenartigen Ansatz 12 mündet.

Am distalen Ende 1 sind die Blattseiten 7, in denen Längsnuten 16 vorgesehen sind, durch einen kreisförmigen Uebergang von der Schmalseite 4 zur Schmalseite 13 abgeschlossen, während sie senkrecht dazu mit relativ grossen Radien in einer Spitze auslaufen (Figur 2). Die Krümmung dieses Auslaufens ist dabei so gewählt, dass möglichst ein stetiger Uebergang des belastenden Kraftflusses vom Schaft 2 auf den umgebenden Zementköcher und/oder das unter Umständen beim Einschlagen der Prothese verdichtete Knochengewebe erfolgt.

Wie Figur 2 erkennen lässt, erweitern sich nicht nur die Blattseiten 7, sondern die Schmalseiten 4 bzw. 13 vom distalen Ende 1 aus konisch, wenn auch der Oeffnungswinkel sehr klein und beispielsweise 0,5-1,5° gegen die Vertikale beträgt. Der Konus der Schmalseiten endet auf dem Niveau 8, das vom distalen Ende 1 etwa 3/4 der Schafthöhe entfernt ist.

Oberhalb dieses Niveaus verlaufen die seitlichen Begrenzungen der Schmalseiten 4 und 13 und damit die Blattseiten 7 parallel zueinander.

Erfindungsgemäss sind mindestens die gegen die Längsmittelachse 3 geneigten Oberflächen der Blattseiten 7 mit Rillen 9 versehen, die sich parallel zur Längsmittelachse und parallel zur Blattoberfläche erstrecken. Bei der gezeigten Ausführungsform sind wegen der einfacheren Herstellung die Längsnuten 16 nicht mit Rillen 9 versehen, die andererseits aber auch einen Teil der oberen parallelen Flächen der Blattseiten 7 bedecken ; selbstverständlich ist es möglich, die geschilderten Wirkungen der Rillen 9 zu verstärken, indem auch in die Längsnuten 16 Rillen 9 eingearbeitet werden. Weiterhin kann ohne ins Gewicht fallende Minderung der verbesserten Haftung der obere Teil der Blattseiten 7 frei von Rillen 9 gehalten werden.

Wie bereits erwähnt, haben sich für Hüftgelenkprothesen Rillenabstände a (Figur 4) von 0,5 bis 2 mm und Rillentiefen t (Figur 4) von 0,2 bis 0,5 mm als besonders wirksam erwiesen.

Die Herstellung der Rillen 9 kann bei metallischen Schäften, die aus den für Gelenkendoprothesen üblichen Metallen oder Metall-Legierungen hergestellt werden, beispielsweise durch Fräsen erfolgen.

**Patentanspruch**

Gerader, blattartiger Schaft (2) für eine Gelenkendoprothese, insbesondere eine Hüftgelenkprothese, dessen Blatt sich vom distalen freien Ende zunächst symmetrisch zu einer Längsmittelachse allseitig konisch erweitert, wobei der Konus der beiden Schmalseiten (4, 13) und die laterale Erweiterung der Blattseiten (7) auf etwa 3/4 der Schafthöhe enden, während medial die Erweiterung der Blattseiten (7) aus dem Konus heraus in einer stetig gekrümmten Kurve in einen, das Schaftblatt vom Prothesenhals (6) trennenden Kragen (12) mündet, dadurch gekennzeichnet, dass die Blattseiten (7) des Schaftes (2) im Bereich des Konus mindestens teilweise mit parallel zur Längsmittelachse (3) und zur Oberfläche der Blattseiten (7) verlaufenden Rillen (9) bedeckt sind, während die Schmalseiten (4, 13) glatte Oberflächen haben, wobei der Rillenabstand (a) 0,5 bis 2 mm und die Rillentiefe 0,2 bis 0,5 mm betragen.

**Claim**

A straight blade-like stem (2) for a joint endoprosthesis, more particularly a hip joint prosthesis, the blade of the stem initially widening conically in all directions from the distal free end symmetrically of a longitudinal centre-axis, the cone of the two narrow sides (4, 13) and the lateral widening of the blade sides (7) terminating about three-quarters of the way along the stem, while medially the widening of the blade sides (7) out from the cone in a continuous curve terminates in a collar (12) separating the stem blade from the prosthesis neck (6), characterised in that the blade sides (7) of the stem (2) are formed in the cone region at least to some extent with grooves (9) parallel to the longitudinal centre-axis (3) and to the surface of the blade sides (7), while the narrow sides (4, 13) have plain surfaces, the between-centres distance (a) of the grooves being from 0.5 to 2 mm and groove depth being from 0.2 to 0.5 mm.

**Revendication**

Tige (2) rectiligne en forme de spatule pour une endoprothèse articulée, en particulier une prothèse coxo-fémorale, dont la spatule s'évase tout d'abord tronconiquement de toutes parts depuis l'extrémité libre distale, symétriquement par rapport à un axe médian longitudinal, le cône des deux côtés étroits (4, 13) et l'évasement latéral des côtés (7) de la spatule s'achevant environ aux 3/4 de la hauteur de la tige tandis que, du côté médial, l'évasement des côtés (7) de la spatule se prolonge au-delà du cône, selon une courbe d'incurvation constante, par une collerette (12) séparant la spatule de la tige d'avec le col (6) de la prothèse, caractérisée par le fait que les côtés (7) de la spatule de la tige (2) sont garnis, au voisinage du cône, au moins partiellement de striures (9) s'étendant parallèlement à l'axe médian longitudinal (3) et à la surface des côtés (7) de la spatule, tandis que les côtés étroits (4, 13) présentent des surfaces lisses, l'espacement (a) entre les striures étant compris entre 0,5 et 2 mm et la profondeur des striures mesurant entre 0,2 et 0,5 mm.

*Fig.2*

*Fig.1*

*Fig.3*

*Fig.4*

1